(19) Europäisches Patentamt
European Patent Office
Office européen des brevets

(11) Publication number : **0 544 612 A2**

(12) **EUROPEAN PATENT APPLICATION**

(21) Application number : **92610083.5**

(51) Int. Cl.$^5$ : **A61K 39/39,** A61K 47/14

(22) Date of filing : **24.11.92**

(30) Priority : **25.11.91 JP 309347/91**
**22.10.92 JP 284458/92**

(43) Date of publication of application :
**02.06.93 Bulletin 93/22**

(84) Designated Contracting States :
**AT BE CH DE DK ES FR GB GR IE IT LI NL SE**

(71) Applicant : **THE NISSHIN OIL MILLS, LTD.**
**23-1, Shinkawa 1-chome, Chuo-ku**
**Tokyo, 104 (JP)**

(72) Inventor : **SEKI, Kuniharu**
**397 Iwai-cho, Hodogaya-ku**
**Yokohama-shi, Kanagawa (JP)**
Inventor : **UMEMURA, Masashi**
**397 Iwai-cho, Hodogaya-ku**
**Yokohama-shi, Kanagawa (JP)**
Inventor : **HATTORI, Sachiko**
**1-19-238, Mouridai**
**Atsugi-shi, Kanagawa (JP)**
Inventor : **KATO, Keiko**
**2-22-17, Nishiogikita**
**Suginami-ku, Tokyo (JP)**
Inventor : **SAKURADA, Miho**
**Sentoraruhaimu KATO2345 Higashinaganuma**
**Inagi-shi, Tokyo (JP)**
Inventor : **OHYAMA, Katsuhiko**
**1, Nakamaru, Kanagawa-ku**
**Yokohama-shi, Kanagawa (JP)**
Inventor : **NEROME, Kuniaki**
**5-13-11, Hase**
**Kamakura-shi, Kanagawa (JP)**

(74) Representative : **Andersen, Henrik Rastrup et al**
**c/o Plougmann & Vingtoft Sankt Annae Plads 11 P.O. Box 3007**
**DK-1021 Copenhagen K (DK)**

# (54) Composition comprising an immunogen and a triglyceride.

(57) An orally or nasally administered immunogen composition comprising an immunogen capable of immunizing mammals and fats and oils of formula (I) :

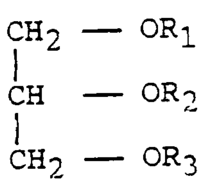

(I)

wherein $R_1$, $R_2$, and $R_3$ respectively mean a $C_{6-24}$ residue of saturated or unsaturated fatty acid, which can exert an enhanced immunization of mammals.

EP 0 544 612 A2

*Fig. 1*

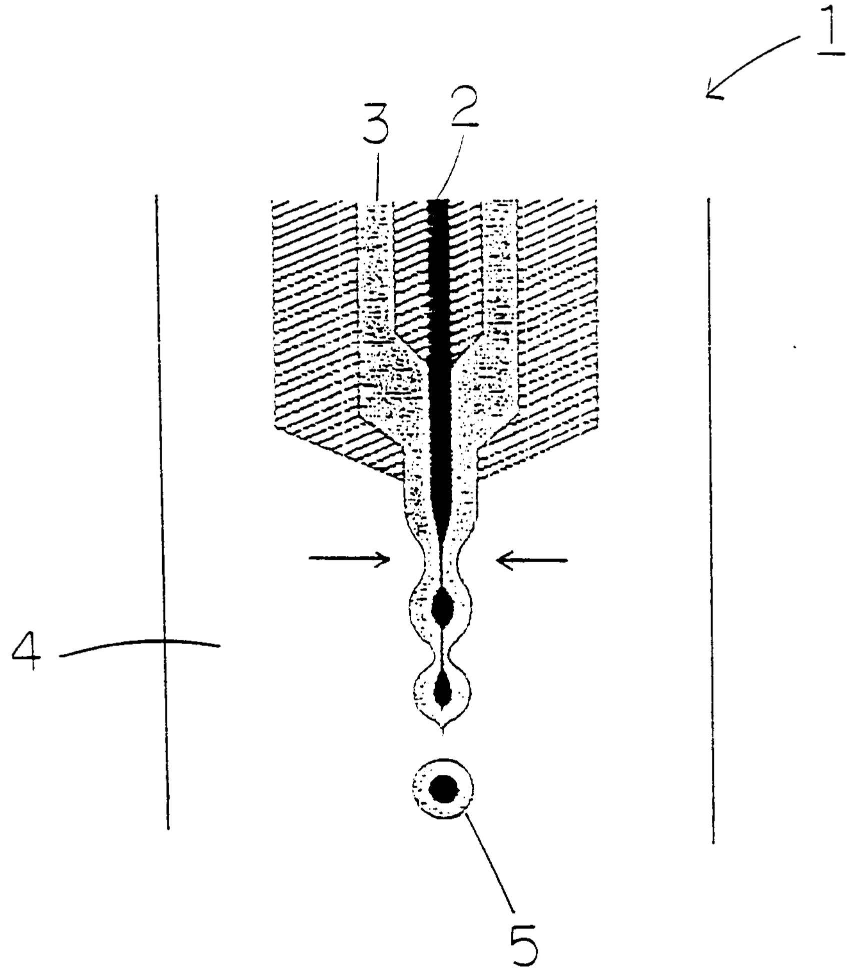

## BACKGROUND OF THE INVENTION

### 1. Field of the Invention

The present invention relates to an immunogen composition for oral or nasal administration.

### 2. Prior Art

Immunogens capable of immunizing mammals are usually suspended in a buffered physiological saline for administration. Such a suspension is referred to as a vaccine. Known vaccines include inactivated vaccines such as a typhoid vaccine, a pertussis vaccine and a rabies vaccine, attenuated vaccines such as a pathogenic micro-organism vaccine and a live polio vaccine, and toxoids such as a diphtheria toxoid and a tetanus toxoid.

Vaccines are usually administered by injection. They are not only inconvenient in administration, but they also tend to cause side effects such as redness, fever, amyotrophy in the administered region.

In order to overcome the above drawback, we have proposed a stable enteric oral administration which is formed a dry spherical body comprising an immunogen and a gelatin (Japanese Laid-Open Patent Publication No. 173829/1991).

On the other hand, by immunoadjuvants is understood an agent for enhancing immunological responses against antigens, which is usually administered with antigens. Immuno adjuvant currently used for human being include aluminum gel such as aluminum hydroxide, aluminum phosphate and alum. Research on immunoadjuvants has made a remarkable progress to develop many immunoadjuvants including synthetic compounds, in addition to bacterial somatic ingredients

## SUMMARY OF THE INVENTION

As the result of extensive study, on immunoadjuvants for orally administrable vaccines, we now formed that a composition comprising an-immunogen and fats and oils can be either orally or nasally administered to enhance the effect of the immunogen.

The present invention provides an immunogen composition for oral and nasal administration, comprising an immunogen capable of immunizing mammals inclusive of human being and a fat and oil of the formula:

$$\begin{array}{lcl} CH_2 & — & OR_1 \\ | & & \\ CH & — & OR_2 \\ | & & \\ CH_2 & — & OR_3 \end{array} \qquad (I)$$

wherein $R^1$, $R^2$ and $R^3$ respectively designate a residue of saturated or unsaturated $C_{6-24}$ fatty acid.

## BRIEF DESCRIPTION OF THE DRAWINGS

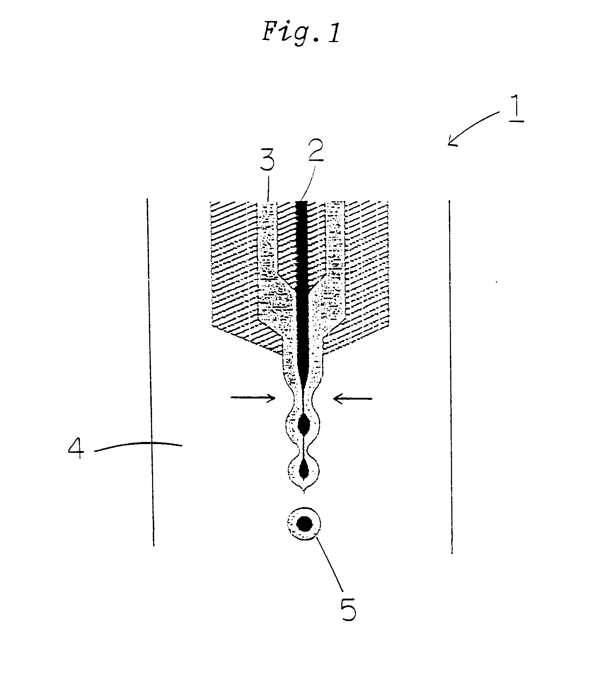

FIG. 1 is a schematic sectional view showing the essential part of a seamless capsule used for manufacturing a double gelatin capsule.

## DETAILED DESCRIPTION OF THE PRESENT INVENTION

According to this invention, "an immunogen capable of immunizing mammals" as recited above which is designates a substance which induces humoral immunity and cellular immunity by producing antibodies and sensitized lymphocytes in mammals. Concrete examples of the substance include everything that can be used for inhibiting infectious diseases, such as vaccines. Vaccines include inactivated vaccines such as an influenza vaccine, a 'Japanese encephalitis vaccine, a pertussis vaccine, a DPT (Diphtheria and Pertussis and Tetanus) vaccine, a cholera vaccine, a pneumococcus vaccine and attenuated vaccines such as a polio vaccine, a paralysis vaccine, a mumps vaccine, a BCG vaccine, a typhoid vaccine and a variola vaccine, toxoids such as a diphtheria vaccine, a tetanus vaccine, a trimeresurus toxoid, a typhoid toxoid and a botulinus vaccine and component vaccines such as an influenza HA vaccine, a hepatitis B vaccine, a hepatitis non-A non-B vaccine, a herpes vaccine, an AIDS vaccine and a cancer vaccine.

Fats and oils used together with said immunogen are triglyceride having a saturated or unsaturated $C_{6-24}$ fatty acid residue of the formula (I). Said fatty acid residues include residues derived from caproic acid, capric acid, lauric acid, myristic acid, palmitic acid, oleic acid, linoleic acid and linolenic acid. These fatty acid residues may be a single residue or a mixture of two or more residues. Fats and oils may be either natural or semisynthetic. Naturally occurring fats and oils include linseed oil, soy-bean oil, olive oil, corn oil, sesame oil, safflower oil, camellia oil, colzaoil, cottonseed oil, peanut oil, palm oil and other edible oils sold on the market. Semi-synthetic oils include MCT (middle chain triglyceride), which designates a $C_{8-12}$ middle chain fatty acid residue contained in triglyceride.

As immunogens used in this invention, a vaccine (in liquid) or its freeze-dried product can be used. Besides, a vaccine (in liquid) which is stabilized in microcapsules made of a gelatin can be also used for the present composition for oral and nasal administration. Further, the microcapsuled immunogen and the freeze-dried vaccine may be dissolved or suspended in an appropriate aqueous medium for administration to be prepared as a suspension or emulsion that can be orally or nasally administered.

Orally administered formulations comprising immunogens and fats and oils include hard gelatin capsules, soft gelatin capsules, tablets, pills, granulations, powders, suspensions and emulsions. Among them, hard gelatin capsules, soft gelatin capsules, suspensions and emulsions are preferable. They can be prepared with a conventional method in the art using immunogens and fats and oils (Process for Preparing Medicaments, Part 1, Published by Chijinkan Publishing House).

Considering the heat stability of the immunogen composition of the present invention, a formulation inhibiting direct contact of immunogens with fats and oils is preferable. However, when a stronger immunological effect is desired, such formulation can be excluded from the consideration. Examples of formulations of immunogen compositions of the present invention include the following three types:

(i) a double gelatin capsule consisting of an external layer and ax internal constituent, said external layer being wormed of a mixture of an immunogen and a gelatin and said inside constituent being formed of fats and oils.

(ii) a capsule charged with an immunogen which is microcapsuled in advance and is subsequently dispersed in fats and oils.

(iii) a suspension or an emulsion by adding a suspending agent or an emulsifier to an immunogen microcapsuled in advance as well as to fats and oils.

A gelatin used for preparing the gelatin capsule or for microcapsuling immunogens preferably have a molecular weight of 80000 to 120000, and a jelly strength of more than 150. Immunogens can be microcapsuled by a conventional method in the art. For example, Japanese Laid-Open Patent Publication No. 173829/ 1991 describes a method for microcapsuling immunogens, which can be used for the present invention. Also available is physical and chemical method such as an interfacial precipitation method precipitating and depositing a natural or artificial high molecular substance on the surface of immuogen particles by dispersing vaccines into a high molecular substance solution and removing the solvent at low temperature under reduced pressure, and a phase separation method producing an immunogen coated with a high molecular substance by dispersing vaccines into a high molecular substance solution and adding a salt and other high molecular substance to make a phase separation. Microcapsules using gelatins provide a stable immunogen composition . Besides, using an appropriate high molecular substance provides an immunogen formulation having desired releasing property.

The immunogen composition of the present invention may be in the form of an enteric preparation. When used as an enteric preparation, preparations such as said microcapsules, capsules and tablets are coated with an enteric film. As enteric materials for forming an enteric film, ones known in the art can be appropriately selected. Preferable examples include methacrylic acid-alkylmethacrylate copolymer, hydroxypropyl methylcellulose phthalate, hydroxypropyl methylcellulose acetate succinate, carbomethyl cellulose, cellulose acetate phthalate, refined shellac, white shellac, ethyl cellulose, aminoalkyl methacrylate polymer. Preparations can be coated with a film coating method using a fluid bed granulator or granulating coating and mixing equipment.

The suspension or emulsion of the present invention can be prepared by suspending or emulsifying directly both immunogens and fats and oils, or microcapsuled immunogen, or either coated or non-coated with an enteric film in fats and oils. In any case of suspending or emulsifying immunogens and fats and oils, a surfactant or additives can be appropriately added there. Surfactants used with a suspension or emulsion include anionic surfactants such as alkyl sulfate, sulfosuccinate and cationic surfactants such as quaternary ammonium salt, nonionic surfactants such as sucrose fatty ester, polyethylene glycol fatty ester, sorbitan fatty ester, propylene glycol fatty ester, polyoxyethylene hydrogenated castor oil, polyoxyethylene sorbitan fatty acid, glycerine fatty ester, polyoxythylene alkyl ether, bloc polymer type and polyoxyethylene alkylaryl ether and both ionic surfactants such as soy bean lecithin. Besides, additives include polyvinyl compounds such as polyvinyl alcohol,

polyvinyl pyrrolidone, hydrophilic high polymers exemplified by cellulose derivatives such as carboxymethyl cellulose, methyl cellulose, carboxyvinyl polymer and cetanol, macrogol, tragacanth, liquid paraffin, and gum arabi.

Formulations of immunogens and fats and oils for nasal administration include liquid preparation such as emulsions which can be formulated using an immunogen and fats and oils with a conventional method using a homogenizer. The emulsion for nasal administration can contain the same, surfactant or emulsifier as mentioned above can be employed together. The emulsion may be preferably employed in the form of drops or aerosols.

The dosage of the composition of the present invention for oral administration depends mainly on the kind of immunogens.

For example, in the case of influenza, the composition is administered usually at a single dose of 50 to 5,000 CCA (Chicken Red Cell Agglutination) for an adult. It may be administered at the same dose two to four weeks after the first administration.

In the case of hepatitis B, the composition is orally administered to one adult usually at a single dose of 0.1 to 10 mg as an antigen protein. It is administered once again four weeks after the first administration, then the third administration 20 to 24 weeks after the first administration. Three doses of the composition are orally administered in total.

In the case of Japanese encephalitis, it is orally administered twice to persons of six month old or more at a dose of 10 to 1,000 μg as an antigen protein at an interval of one to two weeks.

A preferable ratio of the immunogen to fats and oils used together depends on the kind of immunogens; the ratio of 10 to 1,000,000 CCA of the immunogen to 1 g of fats arid oils is preferable in the case of influenza, 0.1 to 10 mg of the immunogen to 1 g of fats and oils in the case of hepatitis B, and 10 to 1,000 μl of the immunogen to 1 g of fats and oils in the case of Japanese encephalitis. The amount of fats and oils used together for adults preferably ranges from 0.1 g to 100 g per day.

The dosage of nasally administered composition depends largely on the kind of immunogens. For example, in the case of influenza, it is nasally administered to one adult at a single dose of 10 to 1,000 CCA, or preferably a single dose of 100 to 500 CCA, and then administered again two to four weeks after the first administration.

Beside, the ratio of the immunogen to fats and oils used together is preferably defined as 10 to 10,000 CCA of the immunogen to 1g of fats and oils in the case of influenza. The amount of fats and oils used together is a single dose of 0.1 to 1,000 mg per one adult, or preferably 0.5 to 500 mg. A dosage of 0.1 mg to 1,000 mg per day is preferable, and then administered again two to four weeks after the first administration.

It is preferable that the composition is formulated in such a manner that a predetermined amount of the immunogen and fats and oils are both contained in a single dose type preparation like a capsule.

The present invention further provides a method for immunizing mammals which comprises administering a pharmaceutically effective amount of the immunogen and an appropriate amount of fats and oils simultaneously to mammals.

## EXAMPLES

The present invention will be fully described with respect to Examples, which are not intended to limit the invention.

### Reference Example 1

In accordance with a method Japanese Laid-Open Patent No. 173829/ 1991 discloses, gelatin 100 g with a molecular weight of approximately 100,000 and a jelly strength of approximately 300 was dissolved in advance in 200 g of 10 mM-phosphate buffered physiological saline (hereinafter referred to as PBS; shown in Table 1) (pH 7.4) to which 400 g (equivalent to 2,400,000 CCA) of an influenza vaccine (A/ Guizhou/54/89 (H3N2)) liquid was added and mixed to formulate a gelatin solution containing the influenza vaccine. Heating this gelatin solution containing the influenza vaccine at 40°C, it was dropped into an oil layer, followed by drying it to give a gelatin sphere containing the influenza vaccine.

[Table 1]

| | | |
|---|---|---|
| Phosphate buffered physiological saline (ph=7.4) | Sodium chloride | 8000 mg |
| | Potassium chloride | 200 mg |
| | Sodium phosphate, dibasic, anhydride | 1150 mg |
| | Potassium phosphate, monobasic | 200 mg |
| Total | | 9550 mg |

The above compounds were soluted in 1 l of distilled water.

Reference Example 2

Gelatin 100 g similar to one used in Reference Example 1 was dissolved in advance in 550 g of 10 mM-PBS (pH 7.4) to formulate a gelatin solution. Heating this solution at 40°C, it is charged in an external layer 3 of a seamless capsule manufacturing machine 1 shown in FIG. 1 while 100 g of either MCT (ODO$^R$: prepared by Nisshin Oil Mills, Ltd. triacylglyceride consisting of 75 % of $C_8$ fatty acid and 25 % of $C_{10}$ fatty acid) or soy bean oil shown in Table 2 or corn oil s(shown in Table 3 prepared by Nisshin Oil Mills, Ltd.) charged in the internal layer 2 thereof. Subsequently, the gelatin solution and oil were dropped into cooled oil 4 by vibrating with ultrasonic wave in the vicinity of the tip of the nozzle of the manufacturing machine. These two-layered liquid flow is converted into particles with interfacial surface tension to form a double gelatin capsule. Then the double gelatin capsule is cooled and dried to give a gelatin capsule containing oil.

[Table2]

| | Soy bean oil |
|---|---|
| Triglyceride (%) | > 99 |
| Phospholipid (%) | 0.003 - 0.045 |
| Unsaponifiable matter(%) | 0.3 |
| sterol | 0.13 |
| tocopherol | 0.11 - 0.18 |
| carbohydrate (squalene) | 0.01 |
| Free fatty acid (%) | < 0.05 |
| Metal (ppm) | |
| iron | 0.1 - 0.3 |
| copper | 0.02 - 0.06 |

[Table 3]

| | corn oil |
|---|---|
| Triglyceride (%) | 79 |
| Sterols (%) | 4.5 |
| Mono-diglyceride (%) | 3.9 |
| Hydrocarbonate and sterol esters (%) | 2.9 |
| Free fatty acid (%) | 1 |
| Polar lipid (%) | 8.7 |

Example 1

Gelatin 100 g similar to one used in Reference Example 1 was dissolved in advance in 200 g of 10 mM-PBS (pH 7.4) to which 400 g (equivalent to 2,400,000 CCA) of an influenza vaccine (A/ Guizhou/54/89 (H3N2)) was added and mixed to formulate a gelatin solution containing the influenza vaccine. The solution maintained at 40°C, is charged in an external layer 3 of a seamless capsule producing machine 1 shown in FIG. 1 while soy bean oil 100 g (prepared by Nisshin Oil Mills, Ltd.) is charged in the internal layer 2 thereof. The resulting double gelatin capsule is coated with an enteric film by use of a granulating coating and mixing equipment (SPIR-A-FLOW: manufactured by Freund Industrial Co., Ltd.). Table 4 shows the formulation of the enteric film liquid for coating the enteric film.

[Table 4]

| | |
|---|---|
| Hydroxypropyl methyl cellulose acetate succinate (HPMC-AS) | 5.2 wt. % |
| Acetylated monoglyceride | 0.5 wt. % |
| Methylene chloride | 47.2 wt % |
| Ethanol | 47.1 wt % |

The enteric film liquid is so formulated that it has an approximately 15 wt% of HPMC-AS. Thus given the enteric gelatin capsule containing influenza vaccine is satisfies the requirement for the disintegration in accordance with an article on enteric preparations in the Disintegration Test Method provided in the Japanese Pharmacopeia.

Example 2

An enteric film was coated on a gelatin sphere containing an influenza vaccine prepared in Reference Example 1 with the same method and the same formulation of the enteric film liquid in Example 1. A gelatin sphere 25 mg (equivalent to 600 CCA of the influenza vaccine) containing the influenza vaccine is distributed in 25 mg of MCT, which is charged in a hard capsule to give an immunogen composition.

Example 3

An enteric film was coated on a gelatin sphere containing an influenza vaccine prepared in Reference Example 1 with the same method and the same formulation of the enteric film liquid in Example 1. A gelatin sphere 25 mg (equivalent to 600 CCA of the influenza vaccine) containing the influenza vaccine is distributed in 25 mg of soy bean oil, which is charged in a hard capsule to give an immunogen composition.

Example 4

An enteric film was coated on a gelatin sphere containing an influenza vaccine prepared in Reference Example 1 with the same method and the same formulation of enteric film liquid in Example 1. A gelatin sphere

25 mg (equivalent to 600 CCA of the influenza vaccine) containing the influenza vaccine is distributed in 25 mg of corn oil, which is charged in a hard capsule to give an immunogen composition.

Example 5

A gelatin sphere 25 mg (equivalent to 600 CCA of influenza vaccine) containing an influenza vaccine prepared in Reference Example 1 is distributed in 25 mg of MCT, which is charged in an enteric coated capsule to give an immunogen composition.

Example 6

A gelatin sphere 25 mg (equivalent to 600 CCA of influenza vaccine) containing an influenza vaccine prepared in Reference Example 1 is distributed in 25 mg of soy bean, which is charged in an enteric coated capsule to give an immunogen composition.

Example 7

A gelatin sphere 25 mg (equivalent to 600 CCA of influenza vaccine) containing an influenza vaccine is distributed in 25 mg of corn oil, which is charged in an enteric coated capsule to given an immunogen composition.

Example 8

Span and tween 20 μl (produced by Nikko Chemical Co. Ltd.) together were added as a sufactant to 5 ml of soy bean oil (prepared by Nisshin Oil Mills, Ltd.) to which 5 ml of influenza vaccine liquids (three kinds of influenza vaccines liquids with different concentrations corresponding respectively to 10,000 CCA, 5,000 CCA, and 2,500 CCA) were further added and mixed well. Subsequently, while cooling the formulation in ice water, it was emulsified with an ultrasonic wave emulsifier (US-150T: manufactured by Nihon Seiki Co., Ltd.) at 40 W for one minute to give an emulsified vaccine.

Example 9

Span (produced by Nikko Chemical Co.,Ltd.) 20 μl was added as a surfactant to 5 ml of soy bean oil (prepared by Nisshin Oil Mills, Ltd.) to which 5 ml of influenza vaccine liquids (three kinds of influenza vaccine liquids with different concentration respectively corresponding to 10,000 CCA, 5,000 CCA and 2,500 CCA were used) were further added and mixed well. Subsequently, while cooling the formulation in ice water, it was emulsified with an ultrasonic wave emulsifier (US-15OT: manufactured by Nihon Seiki Co., Ltd.) at 40 W for one minute to give an emulsified vaccine.

Example 10

Tween (produced by Nikko Chemical Co. Ltd.) 20 μl was added as a surface-active agent to 5 ml of soy bean oil (prepared by Nisshin Oil Mills, Ltd.) to which 5 ml of influenza vaccine liquids (three kinds of influenza vaccines with different concentration respectively corresponding to 10,000 CCA, 5,000 CCA, and 2,500 CCA were used) were further added and mixed well. Subsequently, while cooling the formulation in ice water, it was emulsified with an ultrasonic wave emulsifier (US-150T: manufactured by Nihon Seiki Co., Ltd.) at 40 W for one minute to give an emulsified vaccine.

Example 11

Span and tween (produced by Nikko Chemical Co. Ltd.) 20 μl together were added as a surface-active agent to ODO$^R$ to which 5 ml of influenza vaccine liquids (three kinds of influenza vaccine liquids with different concentration respectively corresponding to 10,000 CCA, 5,000 CCA, and 2,500 CCA were used) were further added and mixed well. Subsequently, while cooling the formulation in ice water, it was emulsified with an ultrasonic wave emulsifier (US-150T: manufactured by Nihon Seiki Co., Ltd.) at 40 W for one minute to give an emulsified vaccine.

Example 12

Span and tween (Nikko Chemical Co. Ltd.) 20 μl together were added as a surfactant to 5 ml of corn oil (manufactured by Nisshin Oil Mills, Ltd.) to which 5 ml of influenza vaccine liquids (three kinds of influenza vaccine liquids with different concentration corresponding to 10,000 CCA, 5,000 CCA, and 2,500 CCA were used) were further added and mixed well. Subsequently, while cooling the formulation in ice water, it was emulsified with an ultrasonic wave emulsifier (US-150T: manufactured by Nihon Seiki Co., Ltd.) at 40 W for one minute to give an emulsified vaccine.

Example 13

Lecithin (produced by Nisshin Oil Mills, Ltd.) 20 μl was added as a surfactant to soy bean oil (prepared by Nisshin Oil Mills, Ltd.) to which 5 ml of influenza vaccine liquids (three kinds of influenza vaccine liquids with different concentration corresponding to 10,000 CCA, 5,000 CCA, and 2,500 CCA were used) were further added and mixed well. Subsequently, while cooling the formulation in ice water, it was emulsified with an ultrasonic wave emulsifier (US-150T: manufactured by Nihon Seiki Co., Ltd.) at 40 W for one minute to give an emulsified vaccine.

Example 14

An influenza vaccine liquid 5 ml was added to 5 ml of soy bean oil (prepared by Nacalai Tesque Inc.) to which already emulsified span and tween (produced by Nikko Chemical Co., Ltd.) were added in the concentration of 4 % (V/V) and the formulation was well stirred. Subsequently, while cooling the formulation in ice water, it was emulsified with an ultrasonic wave emulsifier (US-150T: manufactured by Nihon Seiki Co., Ltd.) at 40 W for one minute to give an emulsified vaccine (which is equivalent to 1,000 CCA/ml of the influenza vaccine).

Example 15

An influenza vaccine liquid 5 ml was added to 10 ml of soy bean oil (prepared by Nacalai Tesque Inc.) to which already emulsified span and tween (produced by Nikko Chemical Co., Ltd.) was added in the concentration of 4 %(V/V) and well the liquid was well stirred. Subsequently, while cooling the formulation in ice water, it was emulsified with an ultrasonic wave emulsifier (US-150T: manufactured by Nihon Seiki Co., Ltd.) at 40 W for one minute to give an emulsified vaccine (which is equivalent to 1,000 CCA/ml of the influenza vaccine).

Example 16

An influenza vaccine 0.96 ml and soy bean oil (prepared by Yoshida Pharmaceutical Co., Ltd.) 0.24 ml were well mixed to be suspended, thereby giving an emulsified vaccine (equivalent to 2,500 CCA/1ml influenza vaccine).

Test Example 1

An gelatin sphere 80 mg containing an influenza vaccine (equivalent to 1,920 CCA as an influenza vaccine) prepared in Reference Example 1 was dissolved in 10 ml of 10 mM-PBS. Subsequently, this gelatin solution 0.25 ml containing influenza vaccine (equivalent to 48 CCA as influenza vaccine) and soy bean oil or MCT 0.05 ml were respectively orally administered to the intestine of ddY mice (10 weeks old) by using a probe. Besides, as a control group, the above-mentioned gelatin solution 0.25 ml containing an influenza vaccine and 10 mM-PBS 0.05 ml were respectively orally administered to the intestine of ddY mice (10 weeks old) by using a probe. The antibody titer of IgG in serum four weeks after the administration was measured with a ELISA method (page 482, Virus Experimentation Written by Hisao Takeda, Published by Maruzen Publishing Co.). The light absorbency was measured with a micro plate reader (manufactured by Corona Co., Ltd.). The wavelength used for the experiment was 415 nm as a primary wavelength and 550 nm as a secondary wavelength. Table 5 shows the results of the measurement.

[Table 5]

| | used with soy bean oil | used with MCT | control |
|---|---|---|---|
| O.D. value (415 nm) | 0.537 | 0.648 | 0.222 |

Test Example 2

An enteric gelatin sphere 25 mg containing a influenza vaccine (equivalent to 600 CCA as an influenza vaccine) coated with an enteric film with a similar method in Reference Example 1 on gelatin sphere produced in Example 1 and a double gelatin capsule containing MCT coated with an enteric film on a double gelatin capsule produced in Reference example 2 were simultaneously orally administered to ddY mice (10 weeks old). Eight doses of the formulation were administered every other day. As a control group, eight doses of only enteric gelatin sphere 25 mg containing influenza vaccine were administered to another group of ddY mice every other day. Four weeks after the administration, the antibody titer of IgG in serum was measured with ELISA method. Table 6 shows the result of the measurement.

[Table 6]

| | 8 doses | control |
|---|---|---|
| O.D. value (415 nm) | 0.509 | 0.020 |

Test Example 3

The emulsified vaccine (1,000 CCA/ml) prepared in Example 14 was administered to ddY mice (seven weeks old, female) at a dose of 1ml per mouse. After 3 weeks of the administration thereof, they are additionally immunized in the same condition to evaluate HI value (hemagglutination-inhibition) assay method (page 217, Virus Experimentation written by Sakae Inoue, Published by Maruzen Publishing Co.). Serum was obtained by extracting blood from fundus oculi every week during the first to fifth week after the first immunization and subjecting it to centrifugal separation process (12,000 rpm, for three minutes). As a control group, an influenza vaccine formulated to 1,000 CCA/ml was orally administered at a dose of 1ml per mouse. Table 7 shows the results of the measurement.

[Table 7]

| | 1 week | 2 week | 3 week | 4 week | 5 week |
|---|---|---|---|---|---|
| oil:vaccine = 1:1 | < 16 | 32 | 64 | 128 | 256 (HI Value) |
| control | < 16 | < 16 | < 16 | < 16 | < 16 |

Test Example 4

The emulsified vaccine (1,000 CCA/ml) prepared in Example 15 was orally administered to ddY mice (seven weeks old, female) at a dose of 1ml per mouse. After three weeks of the administration thereof, they were additionally immunized in the same condition to evaluate HI value in the serum until the fifth week. Serum was obtained by extracting blood from fundus oculi every week during the first to fifth week after the first immunization and subjecting it to centrifugal separation process (12,000 rpm, for three minutes). As a control group, an influenza vaccine formulated to 1,000 CCA/ml was orally adminitered at a dose of 1ml per mouse. Table 8 shows the results of the measurement.

[Table 8]

| | 1 week | 2 week | 3 week | 4 week | 5 week |
|---|---|---|---|---|---|
| oil:vaccine = 2:1 | < 16 | 32 | 128 | 512 | 1024 (HI Value) |
| control | < 16 | < 16 | < 16 | < 16 | < 16 |

Test Example 5

The emulsified vaccine prepared in Example 16 (2,500 CCA/ml) was dropped at the dose of 20 µl into two nostrils of ddY mouse (produced in SLC, Japan; 6 weeks old, female), which is anesthetized by pentobarbital, to administer 40 µl (100 CCA) in total.

After the administration thereof, every week between the first and the third week, serum was obtained by extracting blood from fundus oculi and subjecting it to centrifugal separation process (12,000 rpm, for three minutes). The antibody titer against the influenza vaccine in serum thus given was measured with HI assay method. As a control group, an influenza vaccine was nasal administered at a dose of 40 µl (100 CCA) per mouse. Table 9 shows the results of the measurement.

[Table 9]

| | 1 week | 2 week | 3 week |
|---|---|---|---|
| soy bean oil influenza vaccine emulsion | 32 | 256 | 512 (HI value) |
| control | 32 | 64 | 128 |

Test Example 6

The emulsified vaccine (2,500 CCA/ml) prepared in Example 16 was dropped at a dose of 40 µl into the nostrils of mice like Test Example 5. After extracting the whole blood from mice three weeks after the administration thereof, the thoracic air duct was exposed. The antibody titer of IgA against the influenza vaccine in 1 ml of PBS with which the lung was cleaned was measured with ELISA method. A control group is the same with Test Example 5. Table 10 shows the result of the measurement.

[Table 10]

| | (unit O.D.) |
|---|---|
| | antibody titer |
| soy bean oil influenza vaccine emulsion | 0.624 |
| control | 0.011 |

## Claims

1. An orally or nasally administerable immunogen composition comprising an immunogen capable of immunizing mammals and fats and oils of the formula (I):

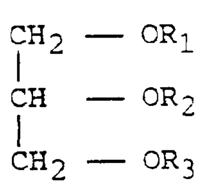

(I)

wherein $R_1$, $R_2$, and $R_3$ respectively mean a residue of saturated or unsaturated $C_{6-24}$ fatty acid.

2. The immunogen composition of claim 1, wherein the immunogen is influenza, Japanese encephalitis, hepatitis B or pertussis.

3. The immunogen composition of claim 1, wherein the fats and oils are soy bean oil, corn oil or middle chain triglyceride.

4. The immunogen composition of claims 1, 2 or 3, which is in the form of a gelatin capsule which has an external layer containing a mixture of the immunogen and gelatin and an internal constituent containing fats and oils.

5. The immunogen composition of claim 4, wherein the gelatin capsule is coated with an enteric film.

6. The immunogen composition of claims 1, 2 or 3, which is in the form of a capsule which contains a gelated immunogen spherical form distributed in the fats and oils.

7. The immunogen composition of claim 6, wherein the gelatin sphere include immunogen is coated with an enteric film.

8. The immunogen composition of claims 1, 2 or 3, which is in the form of a liquid formulation such as suspension or emulsion.

9. The immunogen composition of claim 8, wherein the liquid formulation is used as drops.

10. The immunogen composition of claim 8, wherein the liquid a formulation is used as aerosols.

Fig. 1